# EUROPEAN PATENT SPECIFICATION

(11) **EP 2 882 317 B1**
(45) Date of publication and mention of the grant of the patent: **04.01.2017**
(21) Application number: 13798731.9
(22) Date of filing: 07.08.2013
(51) Int. Cl.: A47C 19/22, A47C 19/20

(54) **WELLNESS CENTER STRUCTURE OF SMALL SIZE**
KLEINE WELLNESSZENTRUMSSTRUKTUR
STRUCTURE DE CENTRE DE BIEN-ÊTRE DE PETITE TAILLE

(30) Priority: 07.08.2012 IT PI20120090
(43) Date of publication of application: 17.06.2015
(73) Proprietor: Baroni, Alfredo, 56040 Lorenzana (PI) (IT)
(72) Inventor: Baroni, Alfredo, 56040 Lorenzana (PI) (IT)
(74) Representative: Celestino, Marco
(86) International application number: PCT/IB2013/056463
(87) International publication number: WO 2014/024148

(56) References cited:
- WO-A1-00/40123
- WO-A1-2008/133496
- CN-Y- 2 580 942
- US-A- 5 343 575
- US-B1- 7 832 031

## Description

### Field of the invention

The present invention relates to the field of wellness centers and, in particular, it relates to a structure for wellness centers that can be installed in a relatively small space.

### Description of the prior art

As well known, wellness centers exist in which a user can relax and regenerate the body by different types of regenerating treatments.

Usually, the regenerating treatments offered by wellness centers are directed to parts of the body that are more subjected to skin problems caused by environmental or aging agents.

For example, treatments exist that provide immersion of the users in baths, or swimming pools, containing water usually at a temperature set between 30 and the 36°C and that are directed to: people affected by arthropathy and need to treat their osteoarticular system, people stressed by the daily life who need to relax and regenerate the body, pregnant women who wish to prepare optimally for the delivery, children who approach the water for the first time, disabled people who require rehabilitation. The water temperature contributes to relax muscles and makes it possible to obtain a relief from pain, for example, muscular contractures or recovery after an accident, or after a condition of intensive training by sports people.

The walls of the swimming pools have delivery nozzles of jets of water that apply a hydromassage action on the user's body. Thermal swimming pools are, furthermore, usually equipped, along the edges, with bars, or handles that the users can grasp.

Once ended a bath in a thermal swimming pool the users can be accommodated in another area of the wellness centre where the presence of jets of water at room temperature allows the body to gradually return to the natural temperature allowing to release the heat accumulated within the bath in the thermal swimming pool.

Other treatments normally offered in a wellness center comprise the application of thermal mud deposited by sulphurous waters.

However, the existing wellness centers provide swimming pools of large size used by a lot of people at the same time and, therefore, do not satisfy the necessary conditions of privacy and discretion necessary to obtain a full relaxation.

In WO00/40123 a structure of bed is described comprising a bath arranged under a bed. The bed is movable between a lowered position, in which it covers the bath and the access into it, and a high position that allows to go into the bath.

### Summary of the invention

It is then a feature of the present invention to provide a structure of wellness center of reduced size, in such a way that it can be installed in a relatively small space, such as a room of a hotel, or a different accommodation, or any civil building, and then for allowing to a user to regenerate the body and relax in a familiar and private environment.

It is then a feature of the present invention to provide a wellness structure of reduced size that can be easily and cheaply installed with respect to prior art solutions.

These and other objects are achieved by a wellness structure, according to the invention, comprising:
- a support structure arranged to support a main bed at a first predetermined height (h1) from a floor of a room in which said structure can be installed, said support structure equipped with supporting side walls defining a wellness grotto, under said main bed, said wellness grotto, equipped with an entry through which a user can pass;
- a secondary bed, or plane support, arranged in said wellness grotto, said secondary bed arranged below said main bed at a second height (h2) from the floor of a room, with h2<h1;
- a treatment bath configured to contain a predetermined amount of water, said treatment bath arranged under said secondary bed, and being slidingly mounted on a frame in such a way to be positioned between a withdrawn position, in which said bath is located under said secondary bed and a protruding position in which the bath protrudes from said secondary bed;
- at least one staircase arranged to vertically connect said floor of said room with said first height in such a way to allow a user to reach said main bed. In particular, it can be comprised of:
- a first staircase;
- a second staircase positioned opposite to said first staircase with respect to said main bed. Preferably, the treatment bath is a hydromassage bath.

In particular, in an exemplary embodiment, at least one stair of the staircase can be slidingly mounted along a guide that is substantially parallel to the longitudinal axis of the main bed. This way, said stair forms a drawer, in order to further optimize the available space. In this case, the drawer can be provided with at least one handle to assist its opening or closing movement.

Advantageously, the secondary bed, or plane support, has an end portion, said end portion protruding from said wellness grotto both when said treatment bath is located in the withdrawn position and when said treatment bath is located in the protruding position, in order to form a seat for a user.

In particular, the end portion can be associated to a backrest, said backrest being of tiltable type and arranged to pass from a substantially vertical position, when said treatment bath is located in the withdrawn position, to a substantially horizontal position, in order to form a massage bed along with said protruding portion, when said treatment bath is located in the protruding position in which it protrudes from the secondary bed.

Advantageously, in the substantially vertical position the backrest is adapted to block the entry of the wellness grotto, whereas in the substantially horizontal position the backrest opens the entry of the wellness grotto allowing the user to enter it.

In an exemplary embodiment at least one among said protruding portion and said backrest has a hole arranged to house, in use, the face of the user. This way, the use as massage bed is assisted.

Advantageously, a tilting device is provided arranged to cause the movement of the backrest from the substantially vertical position to the substantially horizontal position and vice-versa. For example, the tilting device can comprise at least one actuator of pneumatic, or hydraulic type.

In an exemplary embodiment of the invention, in said wellness grotto at least one emitter of light at a predetermined frequency can be installed, in order to provide a chromotherapy treatment to a user on the secondary bed, or in the treatment bath.

Advantageously, in the wellness grotto at least one emitter can be provided of a light selected from the group consisting of:
- an emitter of red light;
- an emitter of orange light;
- an emitter of yellow light;
- an emitter of green light;
- an emitter of blue light;
- or a combination thereof.

Advantageously, furthermore, in said wellness grotto at least one spraying nozzle is provided and arranged to deliver a water flow at a lower temperature, or substantially at the same temperature of the environment.

In an exemplary embodiment a lifting means is also provided that is arranged to move the main bed from a starting height qᵢ to a final height q_{f} with q_{f}>qᵢ from the room floor. In this way, it is possible to raise the height of the entry and, accordingly, assisting the entry in the wellness grotto if the height of the room in which the structure is arranged is not high.

Advantageously, the bath has a base surface made of a soft material and/or of an impermeable material.

In particular, the soft material, can be selected from the group consisting of:
- a polyurethane foam;
- a foam rubber;
- feathers of animals;
- synthetic feathers;
- a combination thereof.

### Brief description of the drawings

The invention will be now shown with the following description of an exemplary embodiment thereof, exemplifying but not limitative, with reference to the attached drawings in which:
- figure 1 diagrammatically shows a perspective view of a structure of wellness center, according to the present invention, in a closed configuration;
- figure 2 diagrammatically shows a perspective view of the wellness center structure of figure 1 in an operative configuration;
- figures 3 and 4 diagrammatically show a perspective view of the wellness center structure of figures 1 and 2 respectively in a configuration of minimum encumbrance and in an operative configuration;
- figures from 5 to 7 diagrammatically show the wellness center structure of figures 3 and 4 in a configuration of minimum encumbrance in a top plan view, an elevational front view and an elevational side view, respectively;
- figures 8 to 10 diagrammatically show the wellness center structure of figures 3 and 4 in an operative configuration in a top plan view, an elevational front view and an elevational side view, respectively;
- figures 11 and 12 respectively show in a overlapped position and in an elongated position a possible exemplary embodiment of a headrest provided for the protruding portion of the massage bed according to the present invention;
- figures 13 to 15 show perspective views of three possible exemplary embodiments of the wellness center structure of reduced size provided by the present invention.

### Detailed description of some exemplary embodiments

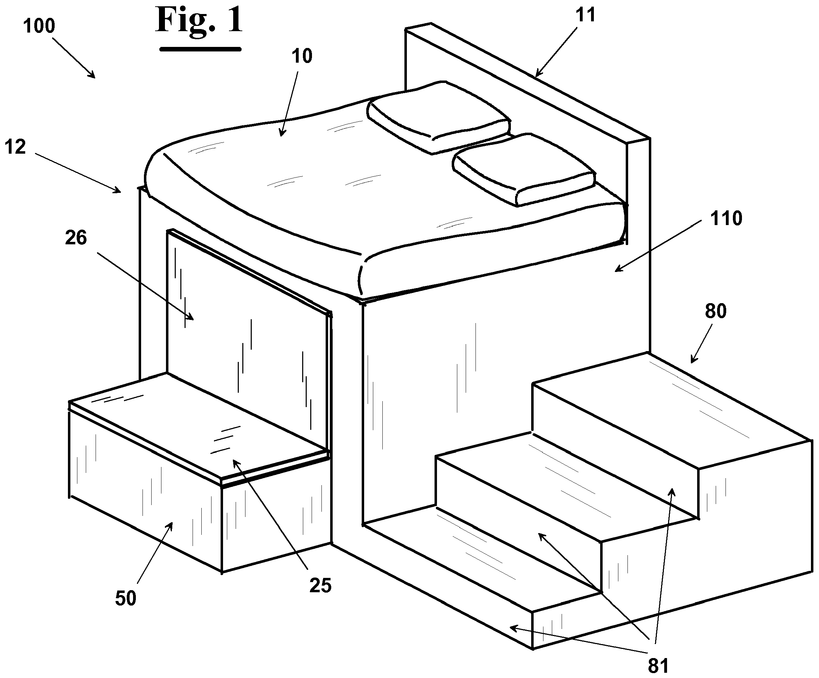

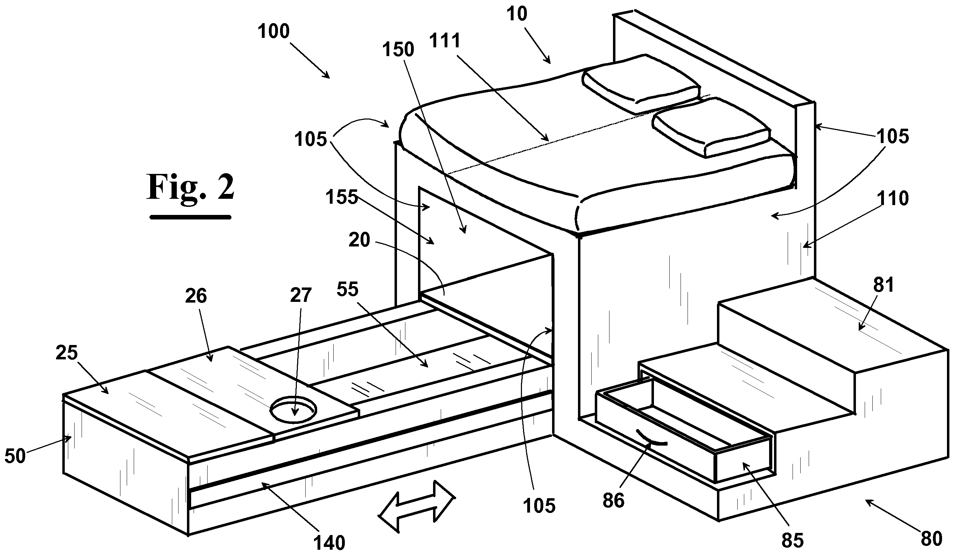

As diagrammatically shown in figures 1 and 2, a wellness center structure 100, according to the invention, comprises a support structure 110 supporting a main bed 10 at a first predetermined height h1 from the floor 200 of a room in which the wellness center 100 can be installed. For example, h₁ can be set between 150 cm and 220 cm. The support structure 110 has supporting side walls 105 defining below said main bed 10 a wellness grotto 150. The wellness center structure 100 is shown in figure 1 in a configuration of minimum encumbrance.

As shown in figure 1, the wellness center structure 100 has a size allowing its installation in a room of an apartment, having a height usually from about 270 cm up to 300 cm. At the same time, the wellness center structure makes it possible to provide treatments like those supplied in a traditional wellness center, with the comfort and the convenience of its positioning in a room of an hotel, or of a civil building, for example a double room of an apartment.

In figure 2 the wellness center structure 100, according to the invention, is shown in an operative configuration.

The grotto 150 has an entry 155 through which a user can pass. The wellness center structure 100, according to the invention, also comprises a secondary bed 20 positioned in the wellness grotto 150 below main bed 10. Secondary bed 20 is arranged at a second predetermined height h₂ from the floor 200 of the room, with h₂, for example, set between 55 and 100 cm.

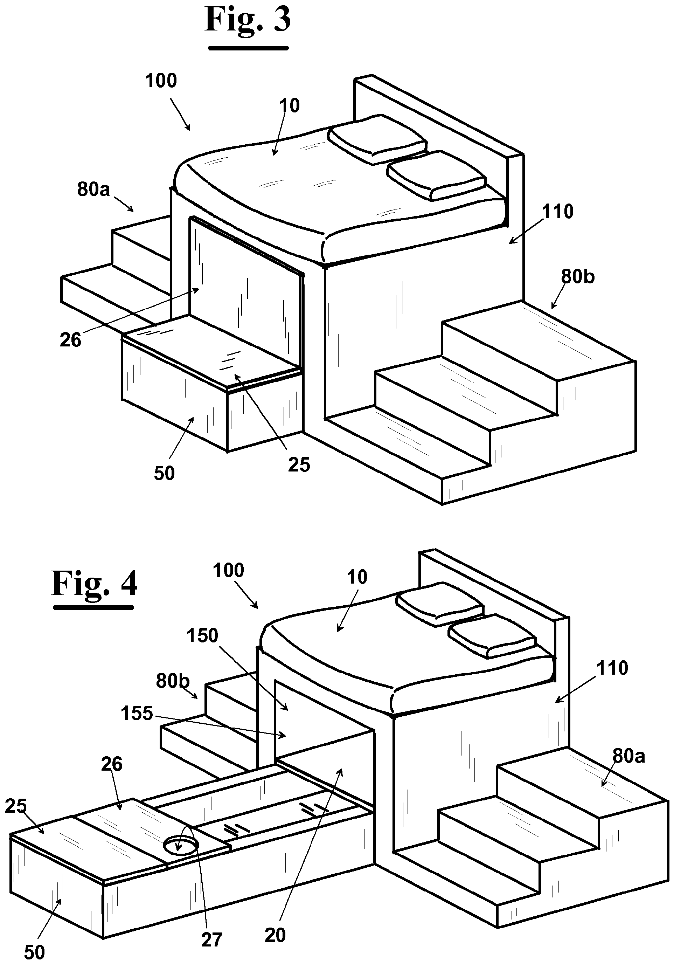

Under secondary bed 20, furthermore, a treatment bath 50 is provided configured to contain a predetermined amount of water. More in detail, the treatment bath 50, for example having a height set between 50 cm and 80 cm, is slidingly mounted on a frame 140 in such a way to pass from a withdrawn position (figures 1, 3 and from 5 to 7), in which the bath 50 is arranged under secondary bed 20, to a protruding position (Figs. 2, 4 and from 8 to 10) in which the bath 50 protrudes from the perimeter of secondary bed 20. More precisely, the bath 50 is slidingly mounted with respect to a longitudinal axis 111 of main bed 10. In this case, the bath 50 slides through the entry 155 of the wellness grotto 150 in such a way to move between the withdrawn position and the protruding position. The bath 50 can be also equipped with a base surface 55, for example, made of soft material to ensure a maximum comfort to a user. In an exemplary embodiment base surface 55 can be made of an impermeable material, for example PVC (polyvinylchloride), of the type used for lining swimming pools, for example including polyurethane foam, or other soft material as foam rubber, or other materials used to produce pillows, to assist comfort for the user.

The structure of wellness center 100 also comprises at least one staircase 80 arranged to connect the floor 200 of the room with the first height h₁ to allow a user to reach main bed 10, and with a second height h2 to allow a user to reach secondary bed 20. In an exemplary embodiment, at least one stair 81 of the staircase 80 can be slidingly mounted along a guide substantially parallel to the longitudinal axis 111 of main bed 10, not shown in the figure for simplicity of illustration. This way, the stair 81 forms a drawer 85 that can be extracted from said staircase 80 and that can be, advantageously, provided with a handle 86 (figure 2).

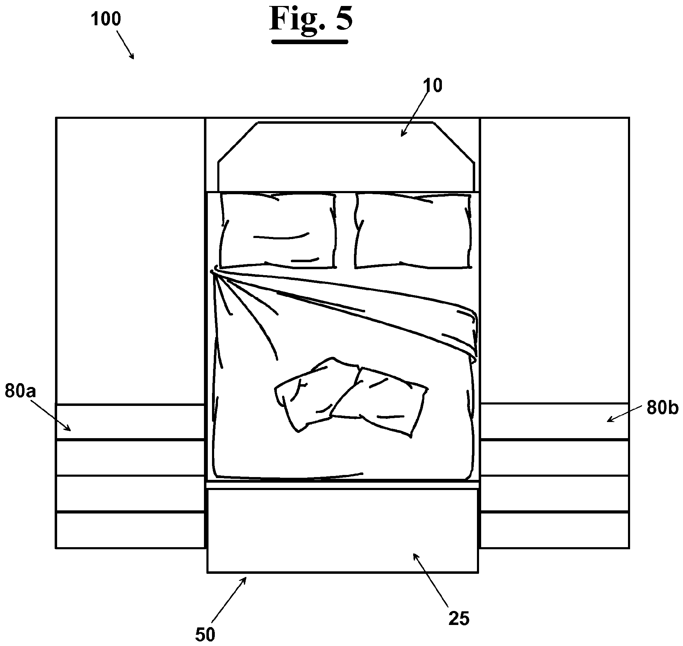

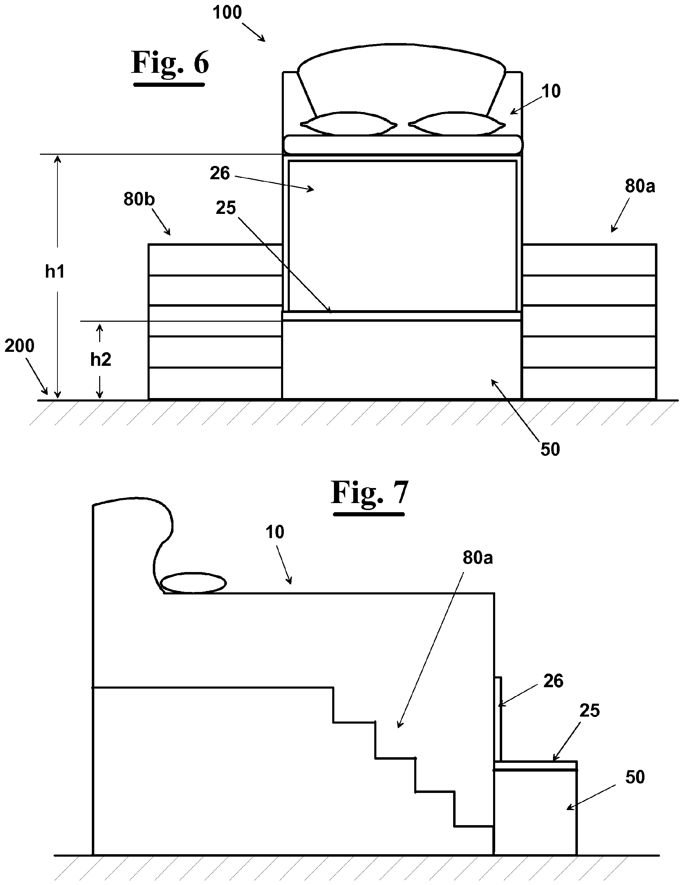

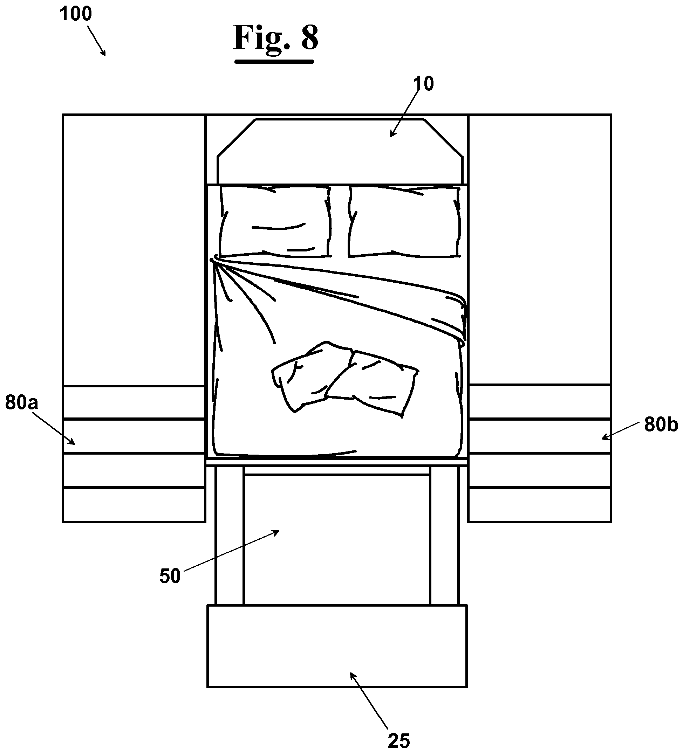

In the exemplary embodiment of figures 3 to 10 two staircases are provided and precisely a first and a second staircase 80a and 80b arranged at opposite sides with respect to main bed 10. Secondary bed 20 can be provided with an end portion 25 that, when the secondary bed, or plane support, 20 is arranged in the withdrawn position (Fig. 1) protrudes from the projection of main bed 10 on the floor, in order to form a seat on which a user can arrange himself/herself.

In the exemplary embodiment of figure 1, the end portion 25 is associated to a backrest 26. This is preferably of tiltable type, in order to be positioned in a substantially vertical position, when the treatment bath 50 is arranged in the withdrawn position (figures 1, 3 and from 5 to 7), or in a substantially horizontal position, in order to form a small bed, for example a massage bed, when the treatment bath 50 is arranged in the protruding position.

In the exemplary embodiment of figures 1 to 10, when the backrest 26 is arranged in the vertical position, it covers the entry 155 of the wellness grotto 150 (figures 1, 3 and from 5 to 7), whereas when the backrest 26 is arranged in the horizontal position the entry 155 of the grotto 150 is free and allows, therefore, a user to enter (figures 2, 4 and from 8 to 10).

More in detail, when the treatment bath 50 is arranged in the protruding position, both main bed 10 and secondary bed 20, as well as the small massage bed 25 and the treatment bath 50, can be used by the user in a small space, like in a room of an hotel, or a bedroom, to have regenerating treatments like those provided in traditional wellness centers. At least one among the protruding portion 25 and the backrest 26 can have a hole 27 arranged to house, in use, the face of the user (figure 2).

In an exemplary embodiment of the invention a tilting device is provided, not shown in the figures, arranged to cause the movement of the backrest 26 from the substantially vertical position to the substantially horizontal position when the treatment bath 50 is brought from the withdrawn position to the protruding position, and to cause the movement of the backrest 26 same from the substantially horizontal position to the substantially vertical position when the treatment bath 50 is brought from the protruding position to the withdrawn position.

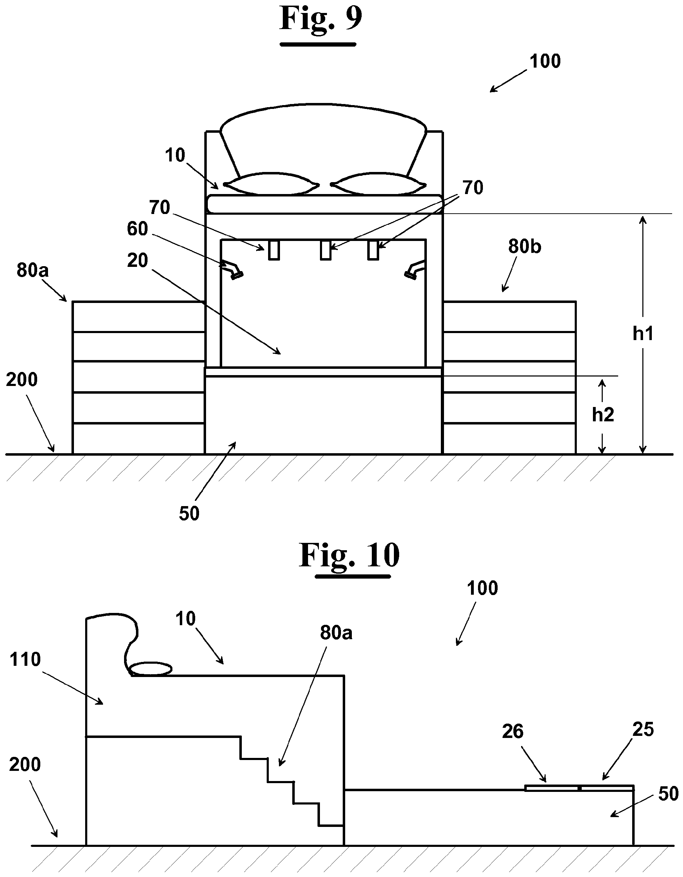

As diagrammatically shown in figure 9, in the wellness grotto 150 at least one emitter 70 of light at a predetermined frequency can be installed, in order to provide a chromotherapy treatment to the user who stays in the grotto, in particular on secondary bed 20.

In addition, or alternatively, in the wellness grotto 150, furthermore, at least one spraying nozzle 60 can be provided arranged to supply a water flow at a lower temperature, or substantially at the same temperature of the environment.

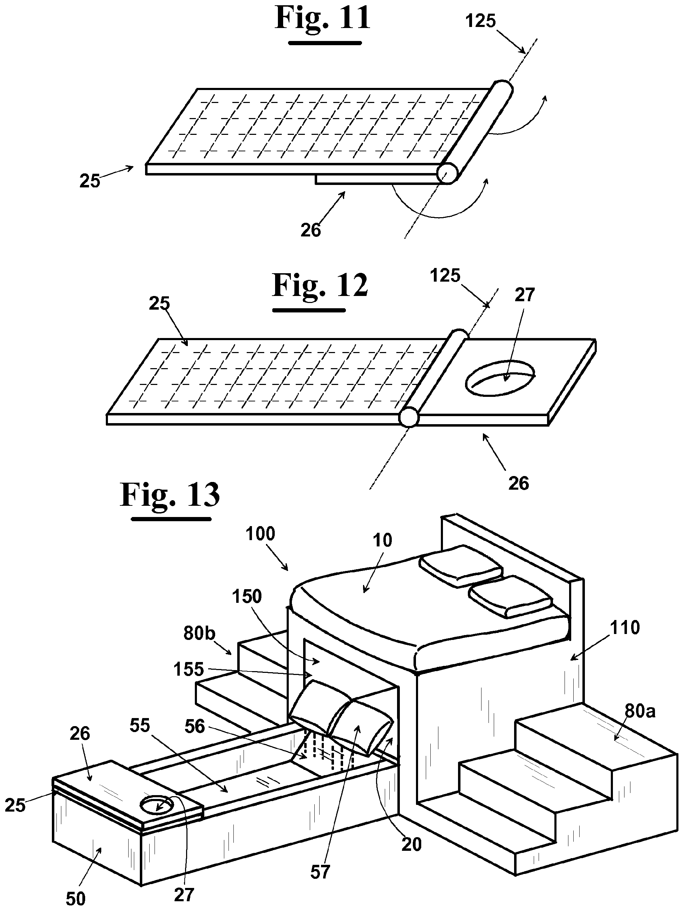

Even though in figure 4, the backrest 26 is shown close to the end portion 25, in the substantially horizontal position, in an alternative exemplary embodiment, as diagrammatically shown in figure 13, in the substantially horizontal position the backrest 26 can be overlapped to end portion 25.

Always as shown in figure 13, the bath 50, for example a hydromassage bath, can be equipped with a surface 56 inclined of a determined angle with respect to a base surface 55. The inclined surface 56 is arranged to house the head and the shoulders of the user arranged in the bath 50 and can be equipped with engagement elements for engaging with at least one pillow 57. For example, the, or each, pillow 57 can be connected to the surface 56 in an adjustable way, for example slidingly mounted on it, in order to be adapted to the size and to the needs of the user.

The end portion 25, for example having a width set between 1.50m and 2.00m can be associated to a headrest 28, diagrammatically shown in figures 11 and 12. More precisely, the headrest 28 can be provided with hole 27 for the face of the user and can be engaged in a removable way to the end portion 25, for example by means of mutual engagement members not shown in the figures, or hinged at the end portion 25, in order to pass from a position, overlapped to the end portion 25 (figure 11), to an elongated position, in which it protrudes from the end portion 25 (figure 12) simply rotating about a rotation axis 125. For example, in the overlapped position the headrest 28 can be arranged under the end portion 25 in order not to block a user that wishes stay on it.

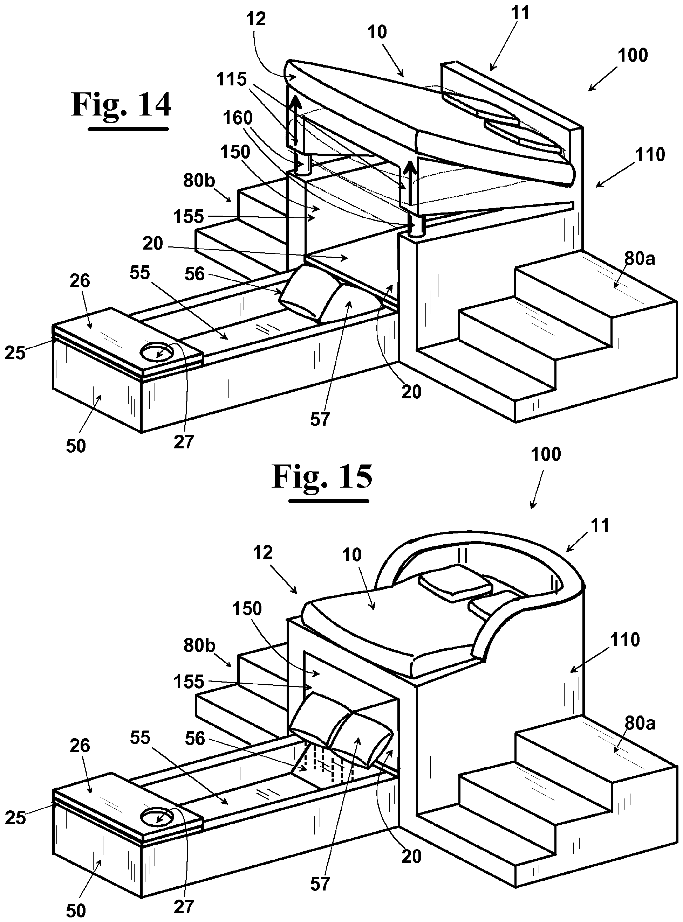

As diagrammatically shown in Fig. 14, the support structure 110 can be equipped with a lifting means that is arranged to move main bed 10 from a starting height qᵢ, to a final height q_{f} with q_{f}>qᵢ from the floor 200 of the room. This way, if the height of the ceiling of the room is low, it is possible, when main bed 10 is not used, to raise the support structure in such a way to assist the entrance in the wellness grotto 150 through entry 155.

For example, the support structure 110 can be equipped, at the end 12 of bed 10, i.e. opposite to the bedhead 11, with portions that can be lifted 115 in order to incline the bed 10 as shown in figure 14. For example, the portions that can be lifted 115 can be operated between two positions by means of pneumatic or hydraulic actuators 160. The user preferably can reach the massage bed 20 with the head oriented outwards of the grotto 150.

In figure 15 a particular solution is shown which provides that the bed 10 has a bedhead 11 having an encircling shape, in order to ensure a maximum comfort to the user arranged on it. In particular, in this case, the bed can be used as a sofa with bedhead 11 that can be used as a backrest by the user.

The foregoing description of specific exemplary embodiments will so fully reveal the invention according to the conceptual point of view, so that others, by applying current knowledge, will be able to modify and/or adapt in various applications the specific exemplary embodiments without further research and without parting from the invention, and, accordingly, it is meant that such adaptations and modifications will have to be considered as equivalent to the specific embodiments. The means and the materials to carry out the different functions described herein could have a different nature without, for this reason, departing from the field of the invention. It is to be understood that the phraseology or terminology that is employed herein is for the purpose of description and not of limitation.

## Claims

1. Structure of wellness center (100) **characterized in that** it comprises:
- a support structure (110) arranged to support a main bed (10) at a first predetermined height (h1) from a floor (200) of the room in which said structure (100) can be installed, said support structure (110) equipped with supporting side walls (105) defining a wellness area, or grotto (150), under said main bed (10), said wellness grotto (150) equipped with an entry (155) through which a user can pass;
- a secondary bed (20) arranged within said wellness area, or grotto, (150), said secondary bed (20) arranged under said main bed (10) at a second height (h2) from the floor (200) of said room, with h₂<h₁;
- a treatment bath (50) configured to contain a predetermined amount of water, said treatment bath (50) arranged under said secondary bed (20) and being slidingly mounted on a frame (140) in such a way to be positioned between a withdrawn position, in which said bath (50) is arranged under said secondary bed (20) and a protruding position in which said bath (50) protrudes from said secondary bed (20), in particular said bath (50) protruding through said entry (155);
- at least one staircase (80) arranged to vertically connect said floor (200) of said room with said first height (h1) in such a way to allow a user to reach said main bed (10).

2. Structure of wellness center, according to claim 1, wherein at least one stair (81) of said staircase (80) is slidingly mounted along a guide substantially parallel to a longitudinal axis (111) of said main bed (10), in such a way that said stair (81) forms a drawer that can be extracted from said staircase (80).

3. Structure of wellness center, according to claim 2, comprising:
- a first staircase (80a);
- a second staircase (80b) arranged opposite to said first staircase (80a) with respect to said main bed (10).

4. Structure of wellness center, according to claim 1, wherein said secondary bed (20) has an end portion (25), said end portion (25) protruding from said wellness grotto (150), both when said treatment bath (50) is arranged in the withdrawn position and when said treatment bath (50) is arranged in the protruding position, in order to form a seat for a user.

5. Structure of wellness center, according to claim 4, wherein said end portion (25) is associated to a backrest (26), said backrest (26) can be tiltable and arranged to pass from a substantially vertical position, when said treatment bath (50) is arranged in the withdrawn position, to a substantially horizontal position, in order to form a massage bed along with said protruding portion (25), when said treatment bath (50) is arranged in the position protruding from said secondary bed (20).

6. Structure of wellness center, according to claim 5, wherein a tilting device is provided arranged to cause the movement of said backrest (26) from said substantially vertical position to said substantially horizontal position and vice-versa.

7. Structure of wellness center, according to any of the previous claims, wherein in said wellness grotto (150) at least one emitter (70) of a light at a predetermined frequency can be installed, in order to provide a chromotherapy treatment to a user in said secondary bed (20), or in said treatment bath (50).

8. Structure of wellness center, according to any of the previous claims, wherein in said wellness grotto (150) at least one emitter (70) is provided selected from the group consisting of:
- an emitter of red light;
- an emitter of orange light;
- an emitter of yellow light;
- an emitter of green light;
- an emitter of blue light;
- or a combination thereof.

9. Structure of wellness center, according to any of the previous claims, wherein, furthermore, at least one spraying nozzle (60) is provided in said wellness grotto (150) and arranged to supply a water flow at a lower temperature, or substantially at the same temperature of the environment.

10. Structure of wellness center, according to any of the previous claims, wherein at least one among said protruding portion (25) and said backrest (26) has a hole (27) arranged to house, in use, the face of the user.

11. Structure of wellness center, according to any of the previous claims, wherein said end portion (25) is associated with a headrest (28), said headrest (28) having a hole (27) for the face of the user and being hinged to said end portion (25), to pass between a position overlapped to said end portion (25) and an elongated position in which protrudes from the end portion (25), by rotating about a rotation axis (125).

12. Structure of wellness center, according to any of the previous claims, wherein said support structure (110) has a lifting means (160) arranged to move said main bed (10) from a starting height qᵢ to a final height q_{f} with q_{f}>qᵢ from the floor (200) of said room, in order to raise the height of the entry (155) and, therefore, assist the entrance in the wellness grotto (150) if said room in which said structure (100) is arranged has a small height.

13. Structure of wellness center, according to any of the previous claims, wherein said bath (50) has a base surface (55) made of soft material to ensure a maximum comfort to the user that use the bath.

14. Structure of wellness center, according to any of the previous claims, wherein said bath (50) has a base surface (55) made of an impermeable material, in particular said base surface (55) being stuffed with a soft material, selected from the group consisting of:
- a polyurethane foam;
- foam rubber;
- feathers of animals;
- synthetic feathers;
- a combination thereof.

15. Structure of wellness center, according to any of the previous claims, wherein said bath (50) is a hydromassage bath.

## Patentansprüche

1. Struktur eines Wellnesszentrums (100), **dadurch gekennzeichnet, dass** es Folgendes umfasst:
- ein Tragwerk (110), eingerichtet für das Tragen eines Hauptbettes (10) in einer ersten vorgegebenen Höhe (h1) über einem Boden (200) des Raumes, in welchen die genannte Struktur (100) eingebaut werden kann, das genannte Tragwerk (110) ausgestattet mit tragenden Seitenwänden (105), welche eine Wellnessgrotte (150) unter dem genannten Hauptbett (10) abgrenzen, die genannte Wellnessgrotte (150) ausgestattet mit einem Eingang (155), den ein Benutzer passieren kann;
- ein sekundäres Bett (20), angeordnet innerhalb der genannten Wellnessgrotte (150), das genannte sekundäre Bett (20) angeordnet unterhalb des genannten Hauptbettes (10) in einer zweiten Höhe (h2) über dem Boden (200) des genannten Raumes, wobei h₂<h₁ ist;
- ein Behandlungsbad (50), ausgebildet für das Aufnehmen einer vorgegebenen Menge von Wasser, das genannte Behandlungsbad (50) unterhalb des genannten sekundären Bettes (20) angeordnet und auf einem Rahmen (140) in einer solchen Weise gleitbar befestigt, dass es zwischen einer eingezogenen Position, in welcher das genannte Bad (50) unter dem genannten sekundären Bett (20) angeordnet ist, und einer hervorstehenden Position, in welcher das genannte Bad (50) aus dem genannten sekundären Bett (20) hervorsteht, positioniert werden kann, wobei das genannte Bad (50) insbesondere durch den genannten Eingang (155) hervorsteht;
- mindestens einen (1) Treppenaufgang (80), angeordnet, um den genannten Boden (200) des genannten Raums senkrecht mit der genannten ersten Höhe (h1) dergestalt zu verbinden, dass einem Benutzer das Erreichen des genannten Hauptbettes (10) ermöglicht wird.

2. Struktur von Wellnesszentrum nach Anspruch 1, wobei mindestens eine (1) Treppe (81) des genannten Treppenaufgangs (80) gleitbar entlang einer im Wesentlichen parallel zu einer Längsachse (111) des genannten Hauptbettes (10) verlaufenden Führung so angebracht ist, dass die genannte Treppe (81) eine Schublade bildet, welche aus dem genannten Treppenaufgang (80) herausgezogen werden kann.

3. Struktur von Wellnesszentrum nach Anspruch 2, umfassend:
- einen ersten Treppenaufgang (80a);
- einen zweiten Treppenaufgang (80b), angeordnet gegenüberliegend dem genannten ersten Treppenaufgang (80a) im Verhältnis zu dem genannten Hauptbett (10).

4. Struktur von Wellnesszentrum nach Anspruch 1, wobei das genannte sekundäre Bett (20) einen Endabschnitt (25) hat und der genannte Endabschnitt (25) aus der genannten Wellnessgrotte (150) hervorsteht, sowohl wenn das genannte Behandlungsbad (50) in der eingezogenen Position angeordnet ist, wie auch wenn das genannte Behandlungsbad (50) in der hervorstehenden Position angeordnet ist, um einen Sitz für einen Benutzer zu bilden.

5. Struktur von Wellnesszentrum nach Anspruch 4, wobei der genannte Endabschnitt (25) mit einer Rückenlehne (26) verbunden ist, wobei die genannte Rückenlehne (26) neigbar sein kann und so angeordnet sein kann, dass sie aus einer im Wesentlichen senkrechten Position, wenn das genannte Behandlungsbad (50) in der eingezogenen Position angeordnet ist, in eine im Wesentlichen waagrechte Position übergehen kann, um zusammen mit dem genannten hervorstehenden Abschnitt (25) eine Massageliege zu bilden, wenn das genannte Behandlungsbad (50) in der aus dem genannten sekundären Bett (20) hervorstehenden Position angeordnet ist.

6. Struktur von Wellnesszentrum nach Anspruch 5, wobei eine Neigevorrichtung vorgesehen ist, so angeordnet, dass sie die Bewegung der genannten Rückenlehne (26) aus der genannten im Wesentlichen senkrechten Position in die genannte im Wesentlichen waagrechte Position und umgekehrt verursacht.

7. Struktur von Wellnesszentrum nach einem beliebigen der vorhergehenden Ansprüche, wobei in der genannten Wellnessgrotte (150) mindestens eine (1) Strahlungsquelle (70) eines Lichts von einer vorgegebenen Frequenz eingebaut werden kann, um einem Benutzer in dem genannten sekundären Bett (20) oder in dem genannten Behandlungsbad (50) eine Farbtherapiebehandlung zu erbringen.

8. Struktur von Wellnesszentrum nach einem beliebigen der vorhergehenden Ansprüche, wobei in der genannten Wellnessgrotte (150) mindestens eine (1) Strahlungsquelle (70) vorgesehen ist, ausgewählt aus der Gruppe bestehend aus:
- einer Strahlungsquelle von rotem Licht,
- einer Strahlungsquelle von orangefarbenem Licht,
- einer Strahlungsquelle von gelbem Licht,
- einer Strahlungsquelle von grünem Licht,
- einer Strahlungsquelle von blauem Licht,
- einer Kombination derselben.

9. Struktur von Wellnesszentrum nach einem beliebigen der vorhergehenden Ansprüche, wobei darüber hinaus in der genannten Wellnessgrotte (150) mindestens eine (1) Sprühdüse (60) vorgesehen und so angeordnet ist, dass sie einen Wasserstrom bei einer niedrigeren Temperatur als der, oder bei im Wesentlichen derselben Temperatur wie die, Umgebungstemperatur liefert.

10. Struktur von Wellnesszentrum nach einem beliebigen der vorhergehenden Ansprüche, wobei mindestens eine(r) (1) des genannten hervorstehenden Abschnitts (25) und der genannten Rückenlehne (26) ein Loch (27) aufweist, so angeordnet, dass es in Gebrauch das Gesicht des Benutzers aufnimmt.

11. Struktur von Wellnesszentrum nach einem beliebigen der vorhergehenden Ansprüche, wobei der genannte Endabschnitt (25) mit einer Kopfstütze (28) verbunden ist, die genannte Kopfstütze (28) ein Loch (27) für das Gesicht des Benutzers aufweisend und an dem genannten Endabschnitt (25) drehbar angebracht, um durch Drehen um eine Drehachse (125) zwischen einer den genannten Endabschnitt (25) überlappenden Position und einer langgezogenen Position, in welcher sie aus dem Endabschnitt (25) hervorsteht, zu wechseln.

12. Struktur von Wellnesszentrum nach einem beliebigen der vorhergehenden Ansprüche, wobei das genannte Tragwerk (110) eine Hebevorrichtung (160) aufweist, so angeordnet, um das genannte Hauptbett (10) aus einer Starthöhe qᵢ in eine endgültige Höhe q_{f} zu bewegen, wobei q_{f}>qᵢ über dem Boden (200) des genannten Raumes ist, um die Höhe des Eingangs (155) anzuheben und daher das Betreten der Wellnessgrotte (150) zu unterstützen, wenn der genannte Raum, in welchem die genannte Struktur (100) angeordnet ist, eine geringe Höhe aufweist.

13. Struktur von Wellnesszentrum nach einem beliebigen der vorhergehenden Ansprüche, wobei das genannte Bad (50) eine aus weichem Material hergestellte Basisoberfläche (55) aufweist, um dem Benutzer, der das Bad benutzt, maximalen Komfort zu bieten.

14. Struktur von Wellnesszentrum nach einem beliebigen der vorhergehenden Ansprüche, wobei das genannte Bad (50) eine aus undurchlässigem Material hergestellte Basisoberfläche (55) aufweist, welche mit einem weichen Material ausgestopft ist, ausgewählt aus der Gruppe bestehend aus:
- einem Polyurethanschaum,
- Schaumgummi,
- Federn von Tieren,
- synthetischen Federn,
- einer Kombination derselben.

15. Struktur von Wellnesszentrum nach einem beliebigen der vorhergehenden Ansprüche, wobei das genannte Bad (50) ein Hydromassagebad ist.

## Revendications

1. Structure d'un centre de bien-être (100) **caractérisé en ce qu'**il comprend :
- une structure de support (110) agencée pour supporter un lit principal (10) à une hauteur prédéterminée (h1) d'un plancher (200) de la salle dans laquelle ladite structure (100) peut être installée, ladite structure de support (110) étant équipée avec des parois porteuses latérales (105) définissant une cavité de bien-être (150), sous le lit principal (10), ladite cavité de bien-être (150) étant équipée d'une entrée (155) par laquelle peut passer un utilisateur ;
- un lit secondaire (20) disposé au sein de ladite cavité de bien-être (150), ledit lit secondaire (20) étant placé sous le lit principal (10) à une deuxième hauteur (h2) du plancher (200) de ladite salle, où h₂<h₁;
- une baignoire de soins (50) configurée pour contenir une quantité d'eau prédéterminée, ladite baignoire de soins (50) étant agencée sous ledit lit secondaire (20), et montée par coulissement sur un bâti (140) de façon à se positionner entre une position rentrée, dans laquelle ladite baignoire (50) se place sous ledit lit secondaire (20), et une position saillante, dans laquelle ladite baignoire (50) fait saillie dudit lit secondaire (20), en particulier ladite baignoire (50) étant saillante de ladite entrée (155) ;
- au moins un escalier (80) disposé de façon à connecter verticalement ledit plancher (200) de ladite salle avec ladite première hauteur (h1) de façon à permettre à un utilisateur d'atteindre ledit lit principal (10).

2. Structure d'un centre de bien-être selon la revendication 1, au moins une marche (81) dudit escalier (80) étant monté de façon coulissante le long d'un guide substantiellement parallèle à un axe longitudinal (111) dudit lit principal (10), de sorte que ladite marche (81) forme un tiroir pouvant être extrait dudit escalier (80).

3. Structure d'un centre de bien-être selon la revendication 2, comprenant :
- un premier escalier (80a) ;
- un deuxième escalier (80b) disposé face audit premier escalier (80a) relativement audit lit principal (10).

4. Structure d'un centre de bien-être selon la revendication 1, ledit lit secondaire (20) présentant une extrémité (25), ladite extrémité (25) faisant saillie de ladite cavité de bien-être (150), aussi bien lorsque ladite baignoire de soins (50) est agencée dans la position rentrée que lorsqu'elle est agencée dans la position saillante, de façon à former un siège pour un utilisateur.

5. Structure d'un centre de bien-être selon la revendication 4, ladite extrémité (25) étant associée avec un dossier (26), ledit dossier (26) pouvant être incliné et disposé de façon à passer d'une position substantiellement verticale, dans laquelle ladite baignoire de soins (50) est disposée dans sa position rentrée, à une position substantiellement horizontale, de façon à former un lit masseur conjointement avec ladite partie saillante (25), lorsque ladite baignoire de soins (50) est disposée dans sa position saillante depuis le lit secondaire (20).

6. Structure d'un centre de bien-être selon la revendication 5, comprenant un dispositif basculant de façon à causer le déplacement dudit dossier (26) de la position substantiellement verticale à ladite position substantiellement horizontale, et vice versa.

7. Structure d'un centre de bien-être, selon une quelconque des revendications précédentes, dans ladite cavité de bien-être (150) au moins un émetteur (70) d'une lumière, à une fréquence prédéterminée, pouvant être installé, afin de permettre la prestation de soins de chromothérapie à un utilisateur dans ledit lit secondaire (20), ou dans ladite baignoire de soins (50).

8. Structure d'un centre de bien-être selon une quelconque des revendications précédentes, dans ladite cavité de bien-être (150) est placé au moins un émetteur (70) sélectionné parmi le groupe composé :
- d'un émetteur d'une lumière rouge ;
- d'un émetteur d'une lumière orange ;
- d'un émetteur d'une lumière jaune ;
- d'un émetteur d'une lumière verte ;
- d'un émetteur d'une lumière bleue ;
- ou d'une combinaison de ces derniers.

9. Structure d'un centre de bien-être selon une quelconque des revendications précédentes, dans laquelle est placée, en outre, une buse de pulvérisation (60) dans ladite cavité de bien-être (150), disposée pour assurer la fourniture d'un débit d'eau à une température inférieure, ou substantiellement à même température que le milieu.

10. Structure d'un centre de bien-être selon une quelconque des revendications précédentes, dans laquelle au moins un des éléments que sont ladite partie saillante (25) et ledit dossier présente un trou (27) agencé pour contenir, en cours d'usage, la face de l'utilisateur.

11. Structure d'un centre de bien-être selon une quelconque des revendications précédentes, ladite extrémité (25) étant associée avec un repose-tête (28), ledit repose-tête (28) possédant un trou (27) pour la face de l'utilisateur, et étant articulé sur ladite extrémité (25), pour passer entre une position chevauchant ladite extrémité (25) et une position allongée faisant saillie de l'extrémité (25), par la rotation autour d'un axe de rotation (125).

12. Structure d'un centre de bien-être selon une quelconque des revendications précédentes, dans laquelle ladite structure de support (110) possède un dispositif de levage (160) agencé pour déplacer ledit lit principal (10) d'une hauteur initiale qᵢ à une hauteur finale q_{f}, où q_{f}>qᵢ, depuis le plancher (200) de ladite salle, de façon à soulever la hauteur de l'entrée (155) et, par conséquent, à faciliter l'entrée dans la cavité de bien-être (150) si la hauteur de ladite salle dans laquelle est agencée ladite structure (100) est limitée.

13. Structure d'un centre de bien-être selon une quelconque des revendications précédentes, dans laquelle ladite baignoire (50) présente une surface de base (55) réalisée avec une matière souple afin de maximiser le confort de l'utilisateur de la baignoire.

14. Structure d'un centre de bien-être selon une quelconque des revendications précédentes, dans laquelle ladite baignoire (50) présente une surface de base (55) réalisée avec une matière imperméable, en particulier une surface de base (55) bourrée avec une matière souple sélectionnée dans un groupe composé des suivantes :
- une mousse de polyuréthane ;
- mousse de caoutchouc ;
- des plumes naturelles ;
- des plumes synthétiques ;
- une combinaison de ces dernières.

15. Structure d'un centre de bien-être selon une quelconque des revendications précédentes, dans laquelle ladite baignoire (50) est une baignoire d'hydro-massage.
